# EUROPEAN PATENT APPLICATION

(11) **EP 0 913 163 A2**
(43) Date of publication of application: **06.05.1999**
(21) Application number: 98120245.0
(22) Date of filing: 26.10.1998
(51) Int. Cl.: A61M 5/32

(54) **Needle disposal apparatus with reduced odors, enhanced safety and reduced electromagnetic noise**

(30) Priority: 30.10.1997 JP 298758/97
(71) Applicant: Oki Data Corporation, Tokyo 108-8551 (JP); Taiyo Elecs C., Ltd., Kyoto (JP)
(72) Inventor: Ota, Yukio, Oki Data Corp., Tokyo 108-8551 (JP); Akutsu, Naoji, Tokyo 108-8551 (JP); Yanobu, Toshio, Taiyo Elecs Co., Ltd., Kyoto (JP)
(74) Representative: Kirschner, Klaus Dieter, Dipl.-Phys.

(57) **Abstract**

A needle disposal apparatus that melts used hypodermic needles has an entrance chamber, a melting chamber, and a waste box that are joined to form an airtight passage. An air pump draws air through this passage and through an odor filter, thereby removing odors from the melting chamber and waste box. The entrance chamber, melting chamber, and waste box are preferably surrounded by electrically conducting materials that block emission of electromagnetic noise. Power is preferably supplied through parallel switches, which are disabled when the waste box is not installed. The needle is preferably guided through the entrance chamber by a slider with a replaceable syringe guide cap to accommodate different syringe and needle sizes.

## Description

### BACKGROUND OF THE INVENTION

The present invention relates to an apparatus for disposing of used hypodermic needles.

The disposal of used hypodermic syringes, and especially of their needles, is attended with certain risks. The safety of needle disposal has been greatly improved by the development of apparatus that uses electric current to melt the tips of used needles, without requiring the user to remove the needle from the syringe first. Known apparatus of this type has had various problems, however.

Japanese Unexamined Patent Publication No. 40060/1985 disclosed an apparatus that blunted and sterilized the tip of a used needle by causing the tip of the needle to complete a short circuit between a pair of electrodes, then dropped the entire needle into a waste disposal container or waste box. One problem with this apparatus was that it made no provision for the removal of odors caused by, for example, burnt blood in the needle tip. Another problem was that the electrical circuit between the power supply and electrodes included a fuse that could easily blow due to the large flow of current (in excess of one hundred fifty amperes, for example) through the needle tip, allowing an imperfectly sterilized needle to drop into the waste box.

Japanese Unexamined Patent Publication No. 223964/1989 disclosed a somewhat similar apparatus having a small axial-flow fan that drew air from the electrode chamber through an odor filter. This arrangement reduced but did not eliminate the problem of odors, as the efficiency of an axial-flow fan is limited. In particular, odors were not removed from the waste box. The apparatus also lacked a fuse or other type of electrical safety device, creating a possible hazard if a metal object more substantial than a hypodermic needle were to be introduced into the space between the electrodes.

Japanese Unexamined Patent Publication No. 92026/1993 disclosed an apparatus in which the needle was inserted horizontally through a hole in a spring-loaded slider, and was cut, after being softened by electric current, so that only the sterilized tip of the needle dropped into the waste box. This apparatus lacked provisions for odor removal, and the motion of the slider in its guide tube had the undesirable effect of pumping malodorous air out into the surrounding environment. The apparatus could furthermore be operated even when the waste box was not installed, permitting the free escape of odors and red-hot needle tips. Another problem occurred because the slider was normally made of plastic, both for reduced friction against the walls of the guide tube and because plastic sliders can be easily and inexpensively manufactured. The plastic slider provided a large aperture for the emission of electromagnetic noise generated at the instant when the needle established a short circuit between the electrodes. The needle acted as an antenna from which the electromagnetic noise was radiated, interfering with the operation of nearby electronic equipment.

Japanese Utility Patent Publication No. 3008777 disclosed a similar apparatus in which the needle was inserted vertically. No provisions were made for odor removal, but the apparatus attempted to confine odors and sparks by means of a tight-fitting hole. The size and shape of the base ends of hypodermic needles are not standardized, however, and in some cases the base end would not fit snugly into the hole, or would not fit at all.

Japanese Utility Patent Publication No. 3014221 disclosed an apparatus with a partitioned chamber, the electrodes and an aluminized paper waste container being disposed on one side of the partition, and a centrifugal fan being disposed on the other side, facing a catalytic filter in the partition wall. The large volume of the chamber, however, reduced the concentration of odors, hence the efficiency of the catalytic filter in removing them.

A problem in all of the above apparatus was the accumulation of melted metal on the electrodes. U.S. Patent 5,676,859 to one of the present inventors disclosed an apparatus that overcame this problem by means of roller electrodes, and used an air pump of the positive-displacement type to remove odors efficiently from the electrode chamber, but the odor problem in the waste box remained unsolved.

### SUMMARY OF THE INVENTION

It is accordingly an object of the present invention to provide a needle disposal apparatus that maintains a pleasant working environment by removing odors efficiently.

Another object of the invention is to provide a needle disposal apparatus that operates without emitting electromagnetic noise.

Yet another object is to prevent accidents in the operation of a needle disposal apparatus.

Still another object is to provide a needle disposal apparatus that accepts and safely dispose of a variety of different types of needles, including large needles.

According to a first aspect of the invention, a needle disposal apparatus comprises an electrode assembly, a guide assembly, and a waste box. The electrode assembly has a first enclosing portion forming a melting chamber, in which electrodes melt an inserted hypodermic needle. The guide assembly has a second enclosing portion forming an entrance chamber, and means for guiding the hypodermic needle through the entrance chamber into the melting chamber. The waste box, which is disposed below the melting chamber, has an open top for receiving the melted needle, and a vent opening for venting air. The first and second enclosing portions and the waste box are held in tight contact with one another, so that the entrance chamber, melting chamber, and waste box form an airtight passage. An air pump coupled to the vent opening of the waste box draws air through this passage and through an odor filter, thereby removing odors efficiently from both the melting chamber and the waste box. The odor filter is preferably located in the waste box, facing the vent opening.

According to a second aspect of the invention, the entrance chamber is surrounded by electrically conducting covers that act as shields preventing the emission of electromagnetic noise. Alternatively, the first and second enclosing portions of the electrode assembly and guide assembly, the walls of the waste box, and at least part of the means for guiding the hypodermic needle through the entrance chamber are made of electrically conducting shielding materials.

According to a third aspect of the invention, the needle disposal apparatus has a means for detecting the presence of the waste box, and preventing current from being supplied to the electrodes when the waste box is not installed, thereby preventing accidental mis-operation.

According to a fourth aspect of the invention, power is supplied to the electrodes through a plurality of switches that are coupled in parallel, providing a current capacity sufficient for melting even large hypodermic needles. Preferably, the switches have overcurrent detectors, and all of the switches are turned off when excessive flow of current through any one of the switches is detected, thereby providing redundant protection against electrical accidents.

According to a fifth aspect of the invention, the means for guiding the hypodermic needle through the entrance chamber comprises a removable syringe guide cap mounted in a slider, preferably by screwing into the slider. A variety of different syringe guide caps, suitable for both large and small needles, can be mounted in the slider.

### BRIEF DESCRIPTION OF THE DRAWINGS

In the attached drawings:
FIG. 1 is an exterior perspective view of a needle disposal apparatus illustrating a first embodiment of the invention;
FIG. 2 is a left front perspective view of the first embodiment with external covers removed;
FIG. 3 is a right front perspective view of the first embodiment with external covers removed;
FIG. 4 is a right side sectional view of the first embodiment;
FIG. 5 is a perspective view of the roller electrode assembly and motor assembly in the first embodiment;
FIG. 6 is a more detailed right side sectional view of the first embodiment;
FIG. 7 is a right side sectional view of the melting chamber in the first embodiment, illustrating the path of the hypodermic needle;
FIG. 8 is a more detailed right side sectional view of the melting chamber;
FIG. 9 is an external perspective view of the first embodiment with the tray withdrawn;
FIG. 10 is a front sectional view of the tray;
FIG. 11 is a side sectional view of the tray in a partly withdrawn position;
FIG. 12 is a side sectional view of the tray in a fully withdrawn position;
FIG. 13 is a right front perspective view of the tray;
FIG. 14 is a right side sectional view of the tray in a locked position;
FIG. 15 is a right side sectional view of the tray in an unlocked position;
FIG. 16 is a right rear perspective view of the waste box;
FIG. 17 is a right side sectional view of the waste box;
FIG. 18 is a right rear perspective view of the tray in a partly withdrawn position;
FIG. 19 is a right rear perspective view of the tray in the fully withdrawn position;
FIG. 20 is a right front perspective view of the tray, showing the waste box being installed;
FIG. 21 is a plan view of the rear end of the waste box;
FIG. 22 is a sectional view of the air pump;
FIG. 23A is a sectional view of the air pump during an upstroke;
FIG. 23B is a sectional view of the air pump during a downstroke;
FIG. 24 is a schematic diagram of the electrical control system of the first embodiment;
FIG. 25 is a perspective view of the main switch in the first embodiment;
FIGs. 26A, 26B, 26C, and 26D are sectional views of the main switch in various on and off positions;
FIG. 27 is a schematic diagram of the main switch;
FIGs. 28A and 28B are sectional views of the main switch, illustrating the action of the box detection lever;
FIG. 29 is a sectional view of the guide assembly and roller electrode assembly, illustrating the operation of the first embodiment;
FIG. 30 is a sectional view of the guide assembly in a second embodiment;
FIG. 31 is a bottom view of the guide assembly in the second embodiment;
FIG. 32 is a top view of the guide assembly in the second embodiment;
FIG. 33 is a sectional view illustrating the opening of the shutter in the second embodiment;
FIG. 34 is a sectional view of the guide assembly in a third embodiment;
FIG. 35 is an upper perspective view of the slider in the third embodiment;
FIG. 36 is a lower perspective view of part of the slider in the third embodiment;
FIG. 37 is an upper perspective view of the syringe guide cap in the third embodiment;
FIG. 38 is a lower perspective view of the syringe guide cap in the third embodiment; and
FIG. 39 is a perspective view of part of the stopper plate in the third embodiment.

### DETAILED DESCRIPTION OF THE INVENTION

Embodiments of the invention will be described with reference to the attached illustrative drawings, throughout which the same component elements are indicated by the same reference characters.

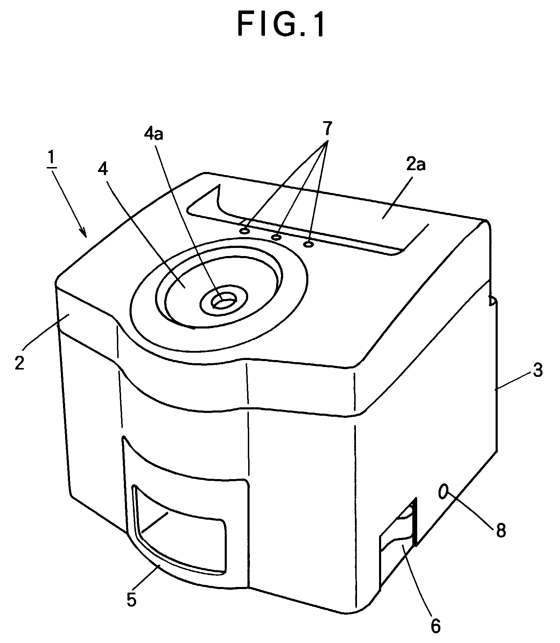

Referring to FIG. 1, the needle disposal apparatus 1 is enclosed by a plastic top cover 2 and a diecast aluminum middle cover 3. The top cover 2 is shaped so as to provide a handle 2a, and has a circular opening for a float 4. The float 4 has a needle insertion hole 4a for insertion of a used hypodermic needle. The middle cover 3 has a front opening for a tray 5, and a side opening for a main switch 6. The needle disposal apparatus 1 has an internal battery, the status of which is indicated by light-emitting-diode (LED) lamps in a row of windows 7 on the top cover 2. An opening 8 is provided next to the main switch 6 for insertion of a battery adapter terminal.

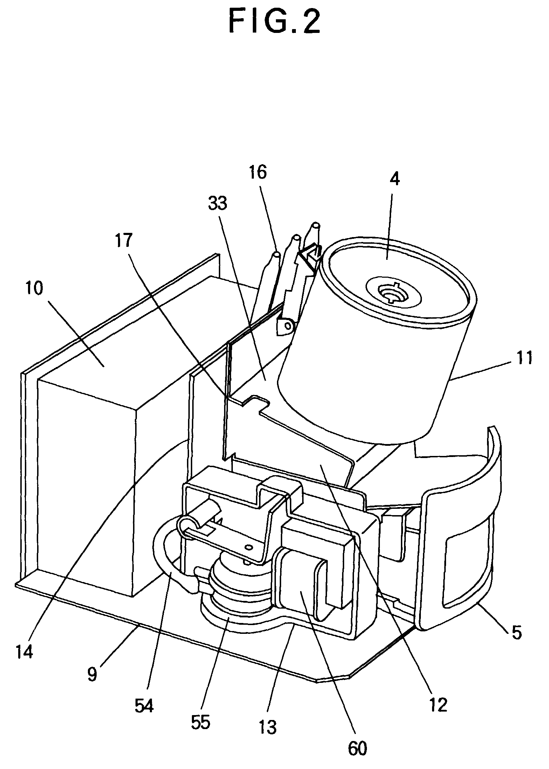

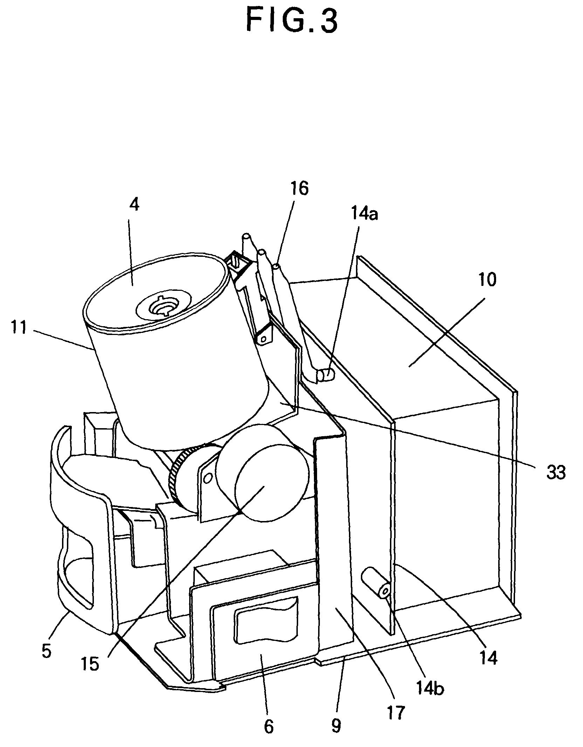

FIGs. 2 and 3 are perspective views of the needle disposal apparatus as seen from the front left and front right, respectively, with the top and middle covers 2 and 3 removed, showing the tray 5, the main switch 6 (FIG. 3), and a base frame 9. Other internal parts of the apparatus visible in these drawings include the battery 10, a guide assembly 11, a roller electrode assembly 12 (FIG. 2), an air pump 13, a control board 14, a motor assembly 15 (FIG. 3), three lenses 16, and a main bracket 17.

The base frame 9 supports the entire needle disposal apparatus. The main bracket 17 is attached to the base frame 9 near its center. Both the base frame 9 and the main bracket 17 are made of electrically conducting materials. The roller electrode assembly 12 is attached to the main bracket 17 at about the middle of the main bracket. The motor assembly 15, which is disposed adjacent the roller electrode assembly 12, is also supported by the main bracket 17.

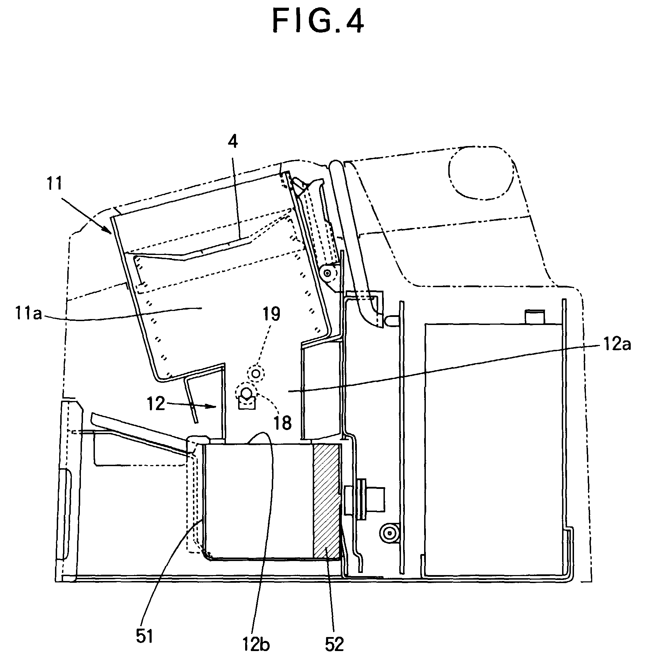

The guide assembly 11 is attached to the main bracket 17 by a supporting bracket 33, located immediately above the roller electrode assembly 12. The tray 5 is disposed below the roller electrode assembly 12, and can be withdrawn to empty a waste box 51, which is shown in the side sectional view in FIG. 4. Also shown in FIG. 4 are two roller electrodes 18 and 19, which melt the tip of an inserted hypodermic needle as described later. Molten metal drops into the waste box 51 through an opening 12b at the bottom of the roller electrode assembly 12.

The air pump 13, which is disposed to the left of the tray 5 in FIG. 2, is a suction pump that draws air from an entrance chamber in the guide assembly 11 through a melting chamber in the roller electrode assembly 12 and out through the waste box 51. Referring again to FIG. 4, the entrance chamber 11a, melting chamber 12a, and waste box 51 are disposed one above another and join to form a closed air passage. No spaces for the escape of odors are left open between the melting chamber 12a and the entrance chamber 11a, or between the melting chamber 12a and the dust box 51.

As shown in FIG. 3, the control board 14 is attached to the rear side of the main bracket 17, and the battery 10 is mounted behind the control board. The control board 14 has three LED lamps 14a that indicate the battery charge status. Light from the LED lamps 14a is guided by the lenses 16 to the windows 17 shown in FIG. 1. The control board 14 also has a battery adapter socket 14b that is aligned with the opening 8 in FIG. 1.

Next, the roller electrode assembly 12 and motor assembly 15 will be described in more detail.

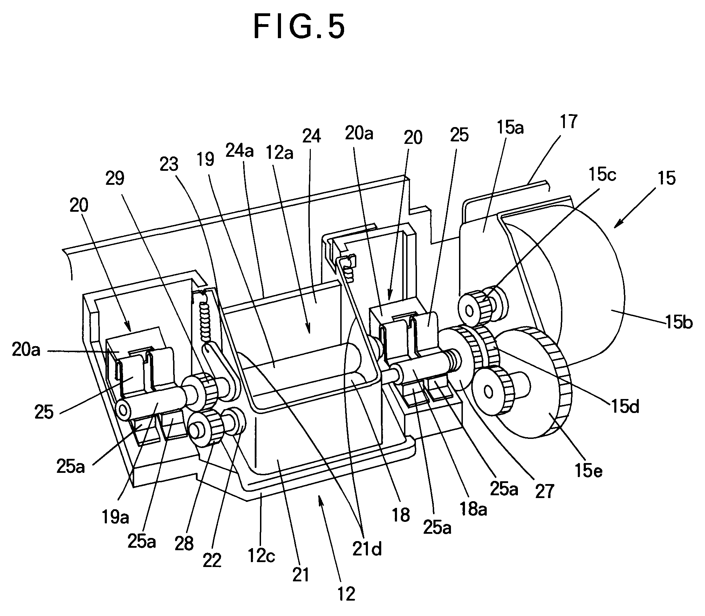

Referring to FIG. 5, the roller electrode assembly 12 comprises the above-mentioned roller electrodes 18 and 19, a pair of brush assemblies 20, an electrode bracket 21, pairs of bushings 22 and 23, a back plate 24, a pair of elastic brush supports 25, a compression spring 26 (not visible), and gears 27, 28, and 29.

The electrode bracket 21, which is U-shaped and made of an electrically conducting material, is attached to an electrode frame 12c, which is made of an electrically non-conducting material. The first pair of bushings 22 are disposed in holes in opposite walls of the electrode bracket 21. The second pair of bushings 23 are disposed in slots in the same opposite walls of the electrode bracket 21. The bushings 22 and 23 are made of an electrically non-conducting material. Only one bushing in each pair is visible in FIG. 5. The holes and slots will be described later.

The back plate 24, which is made of an electrically conducting material, is disposed in the mouth of the U of the electrode bracket 21. The electrode rollers 18 and 19 are enclosed on four sides by the electrode bracket 21 and back plate 24, the enclosed space forming the melting chamber 12a. The upper edges 21d of the electrode bracket 21 and the upper edge 24a of the back plate 24 form a square rim on which the guide assembly 11 is supported, the bottom of the supporting bracket 33 of the guide assembly being held in tight contact with this square rim.

The shaft of the first roller electrode 18 extends through the first pair of bushings 22, and has an enlarged cylindrical part 18a disposed outside and to the right of the electrode bracket 21. The shaft of the second roller electrode 19 extends through the second pair of bushings 23, and has an enlarged cylindrical part 19a disposed outside and to the left of the electrode bracket 21. The enlarged cylindrical parts 18a and 19a are electrically coupled to the surfaces of the roller electrodes 18 and 19, respectively, and comprise a material suitable for making electrical contact with brushes in the brush assemblies 20, so that the brush assemblies can supply respective voltages to the surfaces of the roller electrodes 18 and 19.

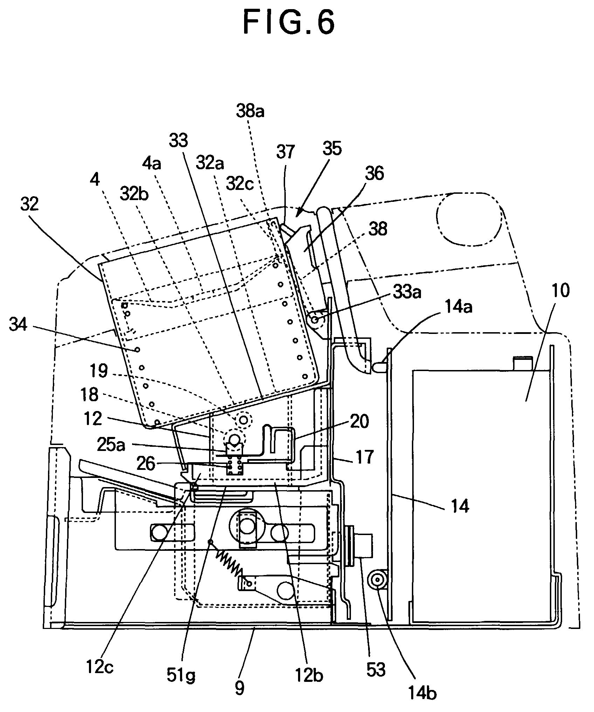

Each brush assembly 20 comprises a support bracket 20a, and a pair of brushes 25a mounted on the brush support 25. The support bracket 20a is disposed on the electrode frame 12c and supports the brush assembly 20 as a whole. One end of the brush support 25 is attached to the support bracket 20a. From this attached end, the brush support 25 is split into two V-shaped parts that hold the brushes 25a. Referring to FIG. 6, the brushes 25a are pressed upward by the compression spring 26, thus establishing electrical contact with the enlarged cylindrical parts 18a and 19a of the shafts of the roller electrodes 18 and 19. The compression spring 26 is held by the electrode frame 12c.

Referring again to FIG. 5, gear 27 is attached to the end of the shaft of the first roller electrode 18, adjacent the enlarged cylindrical part 18a, and is driven by the motor assembly 15, which thereby turns the first roller electrode 18. Gear 28 is attached to the opposite end of the shaft of the first roller electrode 18. Gear 29 is attached to the adjacent part of the shaft of the second roller electrode 19, and meshes with gear 28, thus transmitting the rotation of the first roller electrode 18 to the second roller electrode 19.

The motor assembly 15 comprises a motor bracket 15a, which is attached to the main bracket 17, and a motor 15b. A driving gear 15c is attached to the output shaft of the motor 15b. A gear train comprising further gears 15d and 15e transmits motor torque from the driving gear 15c to the driven gear 27 at the end of the shaft of the first roller electrode 18, with a suitable reduction of rotational speed.

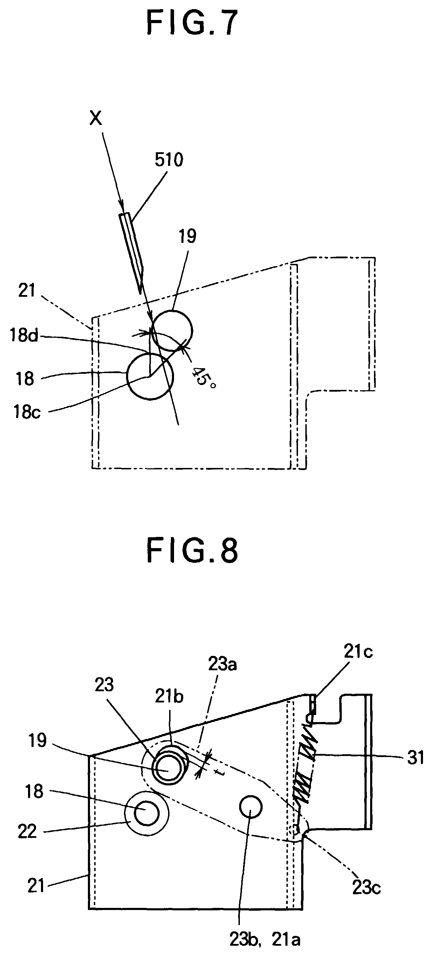

The roller electrodes 18 and 19 are normally held in the positions shown in FIG. 7, in which the second roller electrode 19 subtends an angle of substantially forty-five degrees as seen from the center 18c of the first roller electrode 18. A line drawn from the center 18c of the first roller electrode 18 tangent to the surface of the second roller electrode 19 intersects the surface of the first roller electrode 18 at a point 18d. A hypodermic needle 510 inserted into the needle disposal apparatus enters the space between the roller electrodes 18 and 19 along a path indicated by the letter X, behind the point 18d.

The second roller electrode 19 can be moved by the bushing structure shown in FIG. 8. Bushing 23 has an attached lever 23a that pivots on a pin 23b inserted through a hole 21a in the electrode bracket 21. At one end of this lever 23a, bushing 23 fits into a slot 21b in the electrode bracket 21. A bias spring 31 with hooked ends is stretched between a hole 23c in the other end of lever 23a and a hole 21c in the electrode bracket 21. The bias spring 31 normally holds the bushing 23 and second roller electrode 19 in the position shown in FIG. 7, but when a needle 510 is inserted, the second roller electrode can move by the distance t indicated in FIG. 8 to allow the needle to enter the space between the roller electrodes. The distance t is not large enough to permit the needle 510 to pass freely between the first roller electrode 18 and second roller electrode 19, however. When fully inserted, the needle 510 is forced into contact with the surfaces of both the first roller electrode 18 and the second roller electrode 19, thereby establishing a short circuit.

The needle 510 is guided along path X in FIG. 7 by the guide assembly 11 shown in FIG. 1. Referring again to FIG. 6, the guide assembly 11 comprises a float guide 32, the float 4, the supporting bracket 33, a compression spring 34, and a float detection mechanism 35. The float detection mechanism 35 comprises a switch bracket 36, a limit switch 37, and a movable arm 38.

The float guide 32 has the form of a hollow cylinder which is closed at one end 32a, except for a hole 32b aligned with the needle insertion hole 4a. The closed end 32a of the float guide 32 rests firmly on the supporting bracket 33, which fits tightly against the upper rim of the electrode bracket 21 and the back plate 24, preventing the escape of odors from the melting chamber 12a as noted above.

The compression spring 34 normally holds the float 4 at the top of the float guide 32, in which position the float is detected by the float detection mechanism 35. In the float detection mechanism 35, the movable arm 38 is rotatably mounted on pins in side flanges 33a of the supporting bracket 33, and is pressed toward the float guide 32 by a spring (not visible). The upper end 38a of the movable arm 38 fits into a notch 32c in the upper edge of the float guide 32. When the float 4 is positioned at the top of the float guide 32, the movable arm 38 is forced away from the float guide and actuates the limit switch 37, which is supported by the switch bracket 36, which is attached to the float guide. The presence of the float 4 at the top of the float guide 32 is thereby detected and reported to a control circuit on the control board 14.

Next, the tray 5 and waste box 51 will be described.

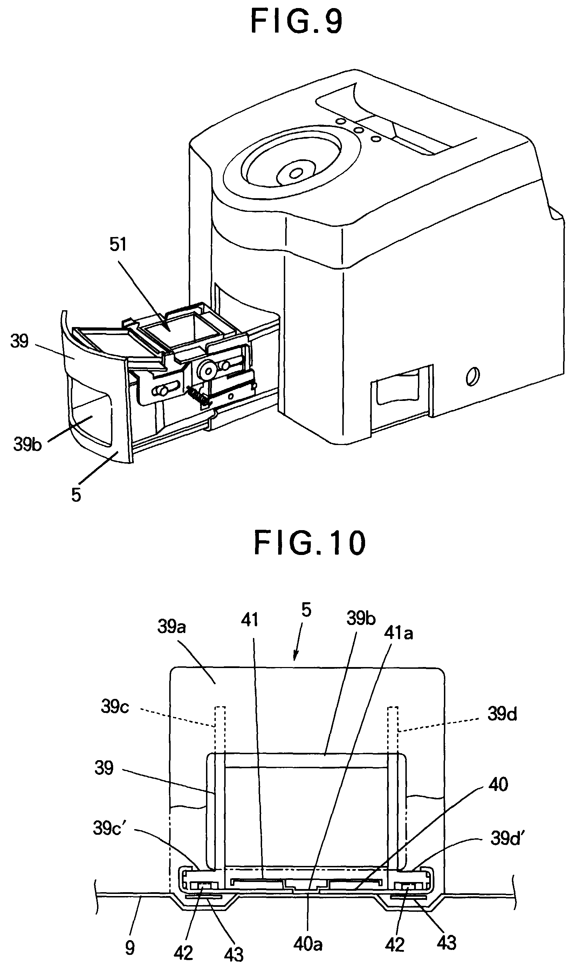

Referring to FIG. 9, the waste box 51 is held in the tray 5 by a mechanism that lowers the waste box when the tray is withdrawn, and lifts the waste box upward against the roller electrode assembly 12 when the tray is closed. The mechanism locks in the closed position, but can be unlocked by means of a finger inserted through a front opening 39b in the frame 39 of the tray 5.

Referring to FIG. 10, the tray frame 39 slides on a sliding bracket 40 and a guide plate 41. The sliding bracket 40 is a rectangular plate with a slot 40a in the middle, and left and right flanged edges that bend upward and inward. The slot 40a extends lengthwise in the sliding direction of the sliding bracket 40. The guide plate 41 is a generally rectangular plate with a pair of stepped cylindrical projections 41a that fit into the slot 40a of the sliding bracket 40. These projections 41a are attached to the base frame 9. The slot 40a permits the sliding bracket 40 to slide between the guide plate 41 and the base frame 9 while the guide plate 41 remains fixed.

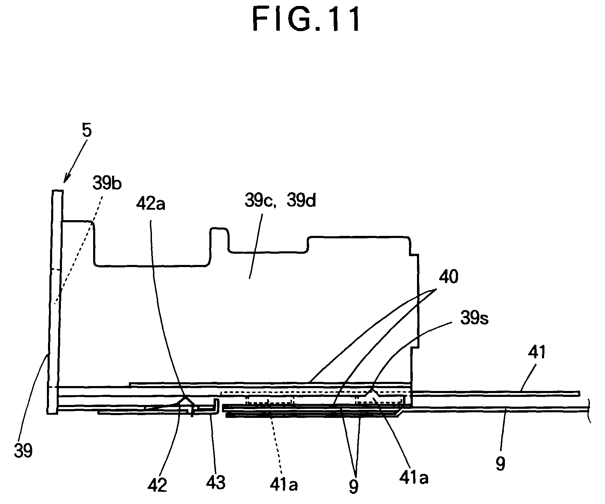

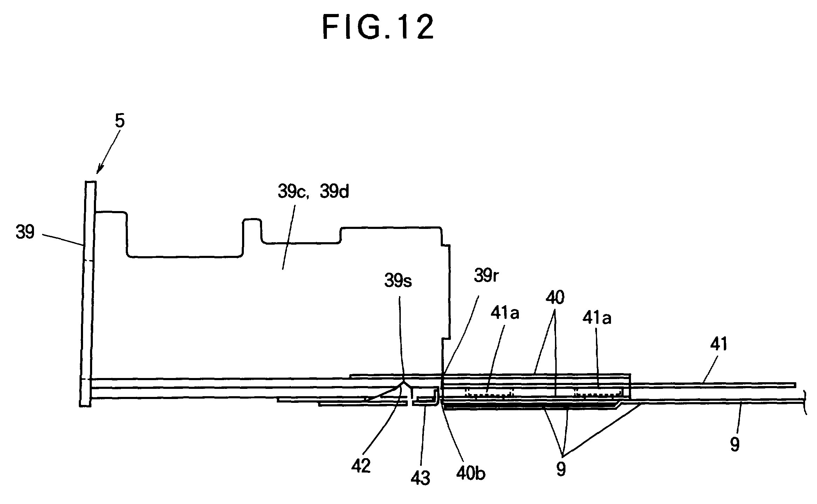

The tray frame 39 has side walls 39c and 39d that terminate at the bottom in outward-extending feet 39c' and 39d'. These feet 39c' and 39d' are loosely held by the flanged edges of the sliding bracket 40, permitting the tray frame 39 to slide with respect to the sliding bracket 40. FIG. 11 shows the tray 5 in a partly withdrawn position. FIG. 12 shows the tray 5 in a fully withdrawn position.

The sliding bracket 40 also has a pair of detent springs 42 and a pair of L-shaped stoppers 43. The L-shaped stoppers 43 prevent the tray 5 from being withdrawn completely from the needle disposal apparatus. The ends of the L-shaped stoppers 43 project upward through openings 40b in the sliding bracket 40, and arrest the motion of the tray frame 39 with respect to the sliding bracket when they abut against the rear wall 39r of the tray frame, as shown in FIG. 12.

The detent springs 42 have bent tips 42a that press upward against the bottom of the tray frame 39. The tray frame 39 does not slide with respect to the sliding bracket 40 unless a force sufficient to overcome the friction of the detent springs 42 is applied. Accordingly, when the tray 5 is pushed or withdrawn, first the tray frame 39 and sliding bracket 40 slide together with respect to the guide plate 41; then the tray frame slides with respect to the sliding bracket.

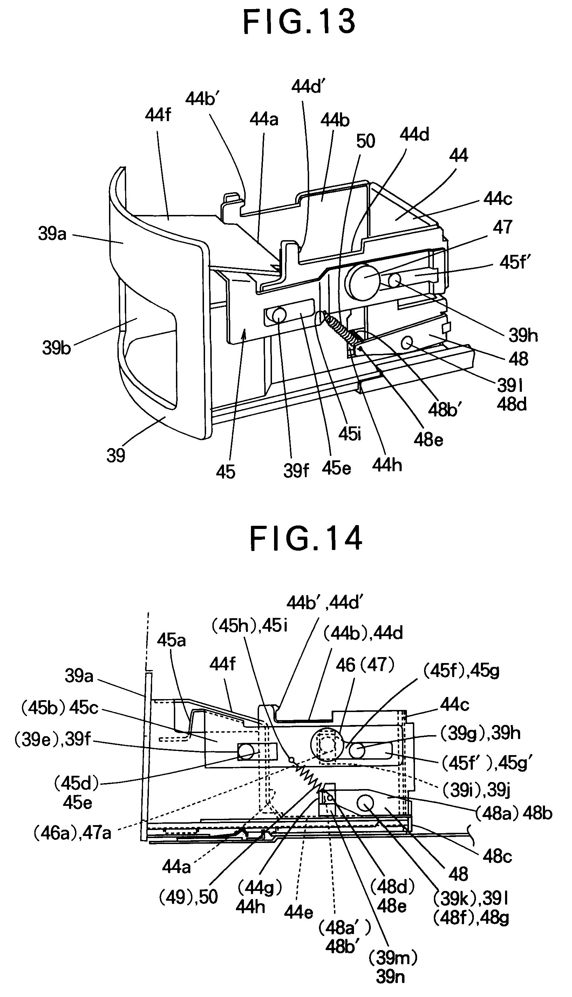

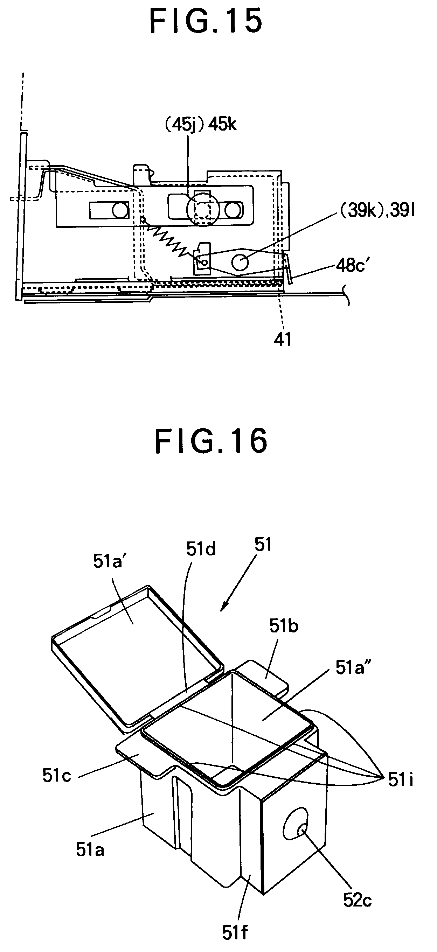

Referring to FIG. 13, the tray 5 also has a waste-box holder 44 that can be raised to the position shown in FIG. 14, and lowered to the position shown in FIG. 15. The waste-box holder 44 is disposed at the rear of the tray frame 39, distant from the front wall 39a of the tray frame. Further components of the tray 5 include a sliding arm 45, best seen in FIG. 13, shoulder collars 46 and 47, indicated in FIG. 14, and a locking arm 48.

Referring to both FIGs. 13 and 14, the waste-box holder 44 has a front wall 44a, a left side wall 44b, a rear wall 44c, a right side wall 44d, a floor 44e, and a shelf 44f extending forward from the top edge of the front wall 44a. The left and right side walls 44b and 44d have respective hook-like projections 44b' and 44d' extending upward from their top front corners.

The sliding arm 45 and locking arm 48 are part of the mechanism that raises and lowers the waste box 51. The locking arm 48 prevents the waste box 51 from being raised when the tray 5 is open, and also prevents the tray from sliding open accidentally.

The sliding arm 45 has a front handle 45a that is bent in a crank-like shape, and left and right side plates 45b and 45c that extend rearwards. These parts are indicated in FIG. 14, although only the front handle 45a and right side plate 45c are visible. The side plates 45b and 45c are bent so as to follow the side walls 39c and 39d of the tray frame 39.

The side plates 45b and 45c of the sliding arm 45 have respective horizontal slots 45d and 45e near their front ends, and respective keyhole slots 45f and 45g near their rear ends. The keyhole slots 45f and 45g include horizontal portions 45f' and 45g' aligned parallel to the horizontal slots 45d and 45e. Spring holes 45h and 45i are disposed near the centers of the side plates 45b and 45c.

The sliding arm 45 slides forward and backward on posts 39e and 39f projecting through the horizontal slots 45d and 45e, and posts 39g and 39h projecting through the horizontal portions 45f' and 45g' of the keyhole slots. Posts 39e, 39f, 39g, and 39h are integral with the side walls 39c and 39d of the tray frame 39. The side walls 39c and 39d of the tray frame 39 also have vertical rectangular slots 39i and 39j aligned with the front portions of the keyhole slots 45f and 45g. The shoulder collars 46 and 47 have respective collars 46a and 46b projecting inward through the keyhole slots 45f and 45g and rectangular slots 39i and 39j. The collars 46a and 46b are attached to the side walls 44b and 44d of the waste-box holder 44.

The locking arm 48 comprises symmetrical left and right locking levers 48a and 48b joined at their rear ends by a crosspiece 48c. Located at the front ends of the levers 48a and 48b are L-shaped extensions 48a' and 48b' and spring holes 48d and 48e. Supporting posts 39k and 39l extend from the side walls 39c and 39d of the tray frame 39 through holes 48f and 48g disposed near the centers of locking levers 48a and 48b, permitting the locking arm 48 to pivot so that the crosspiece 48c moves up and down.

The L-shaped extensions 48a' and 48b' bend inward through vertical rectangular slots 39m and 39n in the side walls 39c and 39d of the tray frame 39. The tips of the L-shaped extensions 48a' and 48b' fit into openings 44g and 44h in the side walls 44b and 44d of the waste-box holder 44, so that the waste-box holder 44 and the front end of the locking lever 48 move up and down together.

A bias spring 49 extends between spring hole 45h in the sliding arm 45 and spring hole 48d in the locking arm 48 on the left side of the tray 5 (not visible). A similar bias spring 50 extends between spring hole 45i and spring hole 48e on the right side of the tray 5. These bias springs 49 and 50 pull the sliding arm 45 toward its rearmost position, as shown in FIG. 14, and pull the front ends of the locking arm 48 upward, causing the crosspiece 48c to move downward. In addition, the L-shaped extensions 48a' and 48b' raise the waste-box holder 44 to its uppermost position.

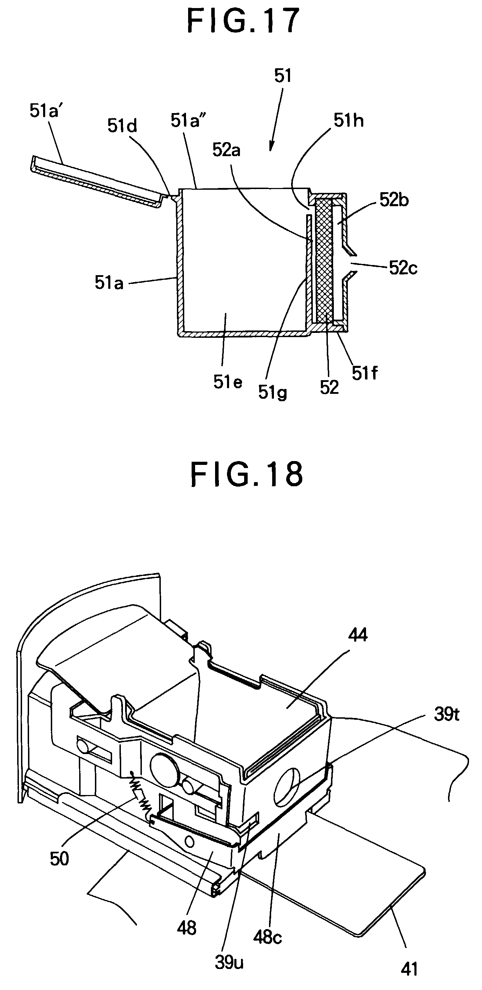

Referring to FIG. 16, the waste box 51 comprises a container 51a with a lid 51a' and side tabs 51b and 51c. The lid 51a' is joined to the container 51a by a thin flexible hinge 51d. The container 51a is partitioned into a waste chamber 51e and a filter chamber 51f. The waste chamber 51e has an open top 51a'' with a raised rim 51i. The waste chamber 51e and filter chamber 51f communicate through an opening 51h situated at a certain height in the partition 51g between them, as shown in FIG. 17.

An odor filter 52 is disposed in the filter chamber 51f, separated by a first space 52a from the partition 51g, and by a second space 52b from the outer wall of the filter chamber 51f, which has a vent opening 52c. When the waste box 51 is mounted in the waste-box holder 44, the position of the vent opening 52c matches the position of an opening 39t in the rear wall of the tray frame 39, shown in FIG. 18. The rear wall 44c of the waste-box holder 44 has a similar opening in the same position.

The operation of loading the waste box 51 into the tray 5 will be described next.

First, to withdraw the tray 5, the user inserts a finger through the front opening 39b in the tray frame 39 (FIG. 13) and pulls forward on the front handle 45a of the sliding arm 45, drawing the sliding arm from the position shown in FIG. 14 to the position shown in FIG. 15. The shoulder collars 46 and 47 are forced downward by the sloping edges 45j and 45k of the keyhole slots 45f and 45g, so the waste-box holder 44 moves downward, while remaining horizontal. The downward motion of the waste-box holder 44 causes the locking arm 48 to pivot on its supporting posts 39k and 39l, so that the crosspiece 48c moves upward and the bottom edge 48c' of the crosspiece rises higher than the guide plate 41. This unlocks the tray 5, so that further pulling on the sliding arm 45 draws the tray to the left in FIG. 15. The pressure of the detent springs 42 brings the sliding bracket 40 forward together with the tray 5, to the position shown in FIGs. 11 and 18.

The bias springs 49 and 50 are designed so that the amount of force required to move the sliding arm 45 from the position in FIG. 14 to the position in FIG. 15 is less than the amount of force needed to draw the tray 5 forward to the position shown in FIG. 11. Thus the user does not need to take a strong grip on the sliding arm 45 or give a strong pull to lower the waste-box holder 44 and raise the crosspiece 48c. The entire withdrawing motion can be accomplished in an easy and natural manner.

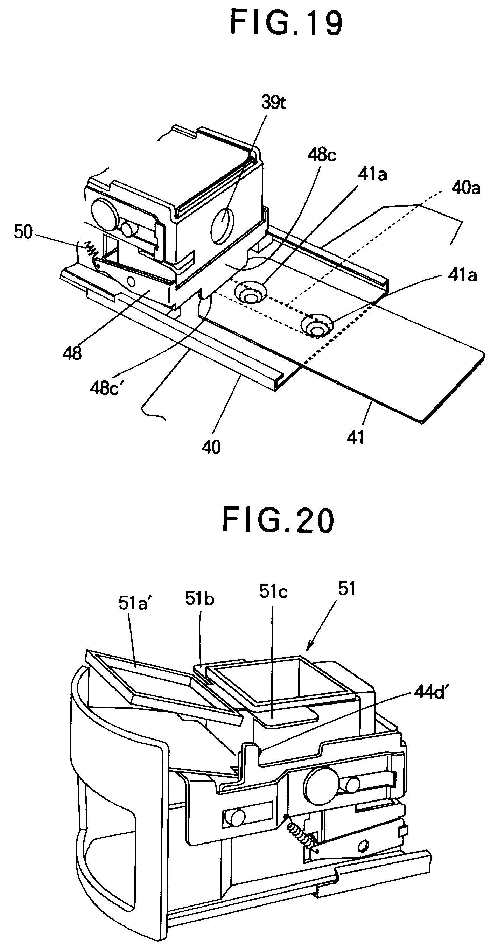

When the tray 5 has been withdrawn a certain distance, the rear end of the slot 40a of the sliding bracket 40 meets the rear stepped cylindrical projection 41a of the guide plate 41, preventing further motion of the sliding bracket 40. Continued pulling on the sliding arm 45 now causes the tray 5 to slide with respect to the sliding bracket 40, overcoming the resistance of the detent springs 42. This sliding motion continues until the rear wall 39r of the tray frame 39 meets the L-shaped stoppers 43, as shown in FIG. 12. The tray 5 cannot be withdrawn past this position. In this position, the detent springs 42 rest in V-shaped depressions 39s in the bottom of the tray frame 39. FIG. 19 is a perspective view of the rear end of the tray 5 in this fully withdrawn position.

After withdrawing the tray 5, the user releases the sliding arm 45. The bias springs 49 and 50 pull the sliding arm 45 backward, but cannot move the locking arm 48, because the crosspiece 48c of the locking arm rests on the guide plate 41. The waste-box holder 44 therefore remains in its lowered position.

Referring to FIG. 20, the user now places the waste box 51 in the waste-box holder 44, with the lid 51a' open. When the waste box 51 is pushed fully down into the waste-box holder 44, the tabs 51b and 51c on the left and right sides of the waste box fit under the hook-like projections 44b' and 44d' on the sides of the waste-box holder, holding the waste box in place.

After loading the waste box 51, the user pushes the tray 5 back into the body of the needle disposal apparatus. At first, the tray frame 39 and sliding bracket 40 move together, because the detent springs 42 are held in the V-shaped depressions 39s in the bottom of the tray frame 39, as shown in FIG. 12. After the tray 5 has been pushed inward a certain distance, the front edge of the slot 40a in the sliding bracket 40 meets the front stepped cylindrical projection 41a of the guide plate 41, preventing further motion of the sliding bracket. The detent springs 42 are then pushed out of the V-shaped depressions 39s, and the tray 5 continues to slide inward while the sliding bracket 40 remains stationary.

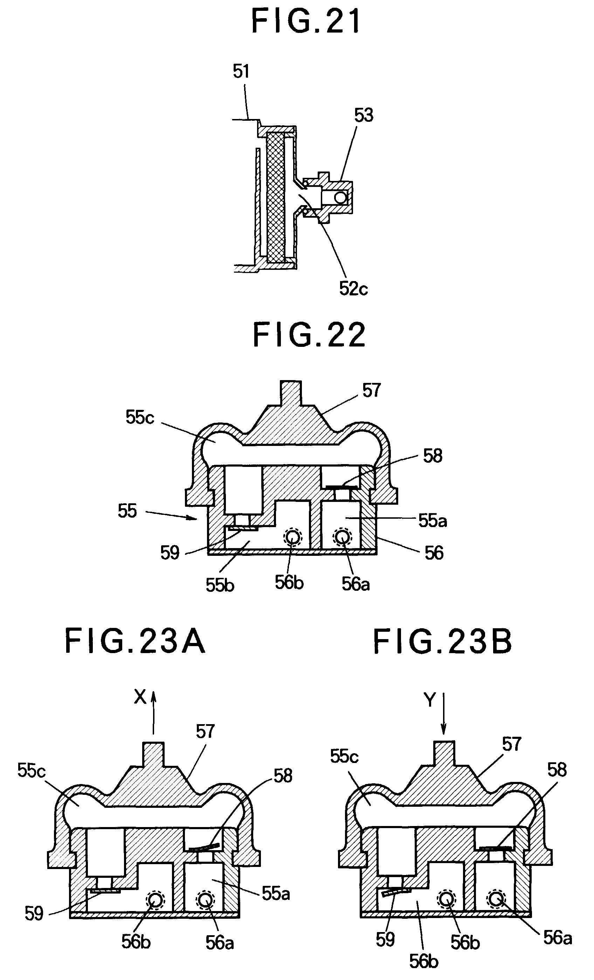

When the tray 5 is pushed fully inward, the crosspiece 48c of the locking arm 48 passes the end of the guide plate 41, and the bias springs 49 and 50 pull the front end of the locking arm upward, so that the crosspiece 48c snaps down and locks the tray 5 in place. This motion of the locking arm 48 also raises the waste-box holder 44, pressing the upper rim 51i of the waste chamber 51e tightly against the bottom of the electrode frame 12c, forming a substantially airtight seal that prevents the escape of odors from the passage formed by the waste box 51 and melting chamber 12a. In this position, the vent opening 52c of the waste box 51, shown in FIG. 17, fits into a suction cap 53 that was shown in FIG. 6. The fit is indicated more clearly in FIG. 21. The suction cap 53 is joined by a tube 54, shown in FIG. 2, to the air pump 13.

Next, the structure of the air pump 13 will be described.

As shown in FIG. 2, the air pump 13 comprises a pump mechanism 55 and an electromagnet 60. Referring to FIG. 22, the pump mechanism 55 has an intake chamber 55a, an exhaust chamber 55b, and a deformable chamber 55c. The walls 56 of the intake chamber 55a and exhaust chamber 55b comprise a rigid material, so that the volume of these chambers 55a and 55b does not change while air is being pumped. An air inlet 56a is formed in the wall of the intake chamber 55a, and an air outlet 56b in the wall of the exhaust chamber 55b. The air inlet 56a is coupled by the above-mentioned tube 54 to the vent opening 52c of the waste box 51.

The deformable chamber 55c is covered by a diaphragm 57 made of a flexible material such as rubber. Upward and downward motion of the diaphragm 57 changes the volume of the deformable chamber 55c. An intake valve 58 permits air to flow from the intake chamber 55a into the deformable chamber 55c, and prevents air from flowing in the reverse direction. An exhaust valve 59 permits air to flow from the deformable chamber 55c into the exhaust chamber 55b, and prevents air from flowing in the reverse direction.

The diaphragm 57 is linked to a reciprocating plunger (not visible) in the electromagnet 60, by which the diaphragm is driven alternately in the direction of arrow X in FIG. 23A and arrow Y in FIG. 23B. In FIG. 23A, as the volume of the deformable chamber 55c expands, air is drawn into the deformable chamber through the air inlet 56a, intake chamber 55a, and valve 58. In FIG. 23B, as the volume of the deformable chamber 55c contracts, the air is forced through valve 59 into the exhaust chamber 55b, and out through the air outlet 56b. Repetition of these motions pumps air from the air inlet 56a to the air outlet 56b.

Next, the electrical control system of the needle disposal apparatus will be described.

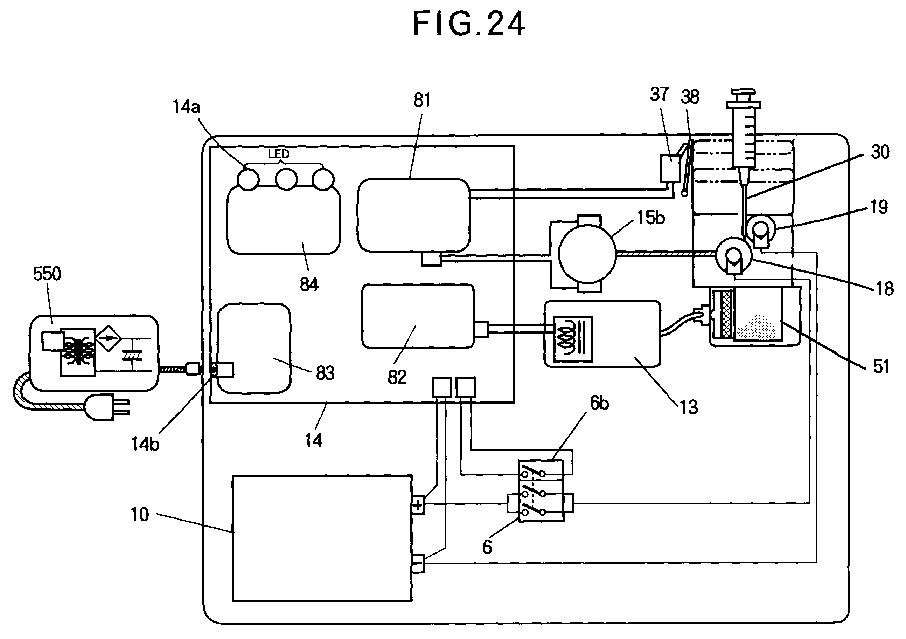

Referring to FIG. 24, the electrical control system comprises the main switch 6, a sub-switch 6b, and various control circuits disposed on the control board 14. The main switch 6 couples the positive terminal of the battery 10 to the first roller electrode 18. The second roller electrode 19 is coupled to the negative terminal of the battery 10. The sub-switch 6b controls the supply of current from the battery 10 to the motor 15b and air pump 13, by way of a circuit that leads from the positive terminal of the battery 10 to the control board 14, then to the sub-switch 6b, again to the control board 14, then to the motor 15b and air pump 13, back again to the control board 14, back to the sub-switch 6b, back once more to the control board 14, and to the negative terminal of the battery 10. These interconnections are indicated in a simplified manner in FIG. 24, which shows only a single pair of interconnecting lines between the control board 14 and sub-switch 6b. The main switch 6 and sub-switch 6b are ganged so that both turn on and off together.

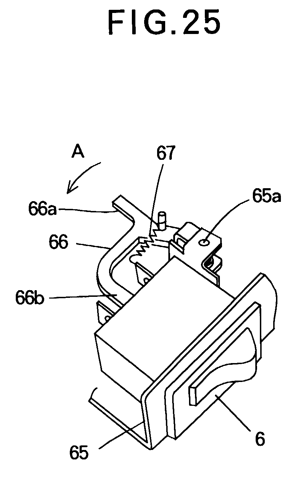

Referring to FIG. 25, the main switch 6 is mounted in a switch bracket 65. One part of the main switch 6 is a box detection lever 66 that pivots on a pin inserted in a hole 65a in the switch bracket 65. The sensing end 66a of the box detection lever 66 is aligned with a slot 39u in the tray frame 39 of the tray 5, shown in FIG. 18, and a similar slot in the waste-box holder 44 (not shown in the drawings). The pushing end 66b of the box detection lever 66 actuates a reset lever in the main switch 6, shown later. When the tray 5 is open, or the tray 5 is closed but the waste box 51 is not installed, a bias spring 67 pulls the main switch 6 in the direction of arrow A in FIG. 25. When the tray 5 is closed with the waste box 51 installed, the sensing end 66a of the box detection lever 66 rests against the side of the waste box 51, preventing this motion of the box detection lever 66.

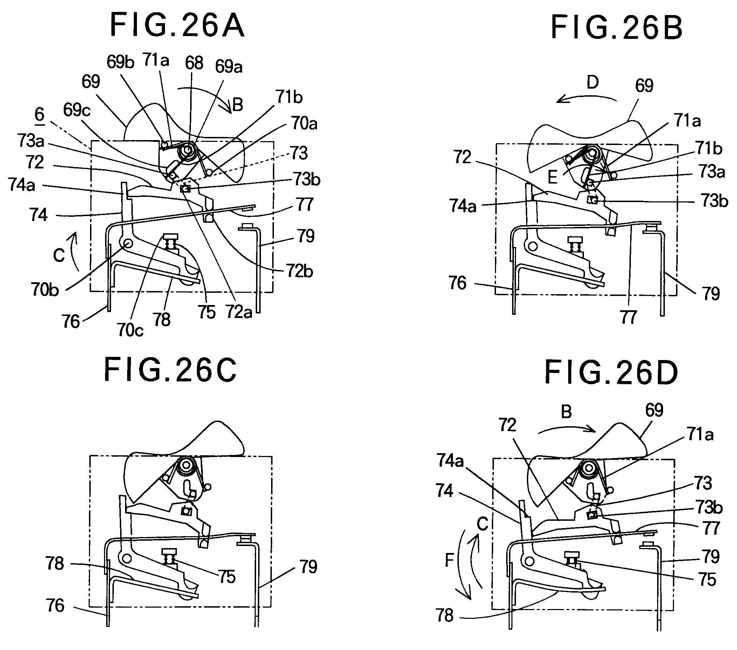

Referring to FIG. 26A, the main switch 6 also has a supporting post 68 on which a switch cap 69 is rotatably mounted, a post 70a against which one end of a torsion spring 71a presses, a post 70b that rotatably supports the above-mentioned reset lever 74, and a restraining ledge 70c that restrains the motion of the reset lever. The torsion spring 71a also presses against a post 69b attached to the switch cap 69, thus urging the switch cap to rotate about its axis 69a in the direction of arrow B, to the position shown, which is the off position of the main switch 6.

The switch cap 69 is coupled to a linking lever 72 by a detent bar 73. A pin 73a at one end of the detent bar 73 fits into an L-shaped slot 69c in the switch cap 69, and is held by a V-shaped plate spring 71b that bends around the axis 69a of the switch cap 69 and presses against the post 69b. A pin 73b at the other end of the detent bar 73 fits into a hole 72a in the linking lever 72. One end of the linking lever 72 is normally seated against a stopper 74a at one end of the reset lever 74. A compression spring 75 disposed between the reset lever 74 and the restraining ledge 70c urges the reset lever 74 to rotate in the direction of arrow C, so that the linking lever 72 remains seated against the stopper 74a.

The input terminal 76 of the main switch 6 is attached to a movable arm 77 and a bimetal bar 78. The movable arm 77 is held in a notch 72b in the linking lever 72. In the off position shown in FIG. 26A, the linking lever 72 is pulled upward by the detent bar 73, preventing the movable arm 77 from making contact with the output terminal 79 of the main switch 6.

Referring to FIG. 26B, to turn on the main switch 6, the user presses the switch cap 69 in the direction of arrow D, overcoming the opposing force of the torsion spring 71a. The plate spring 71b pushes pin 73a of the detent bar 73 toward the lower right in the drawing, causing the detent bar 73 to rotate in the direction of arrow E. Pin 73b is thereby forced downward in the drawing, moving the linking lever 72 in the same downward direction and bringing the movable arm 77 into contact with the output terminal 79, allowing current to flow from the input terminal 76 to the output terminal 79. This contact stops the downward motion of the linking lever 72 and pin 73b of the detent bar 73, forcing pin 73a of the detent bar to move upward in the L-shaped slot 69c, against the force exerted by the plate spring 71b.

Further motion of the switch cap 69 in the direction of arrow D allows pin 73a to return to the lower end of the L-shaped slot 69c, and brings the main switch 6 into the on position shown in FIG. 26C. Due to the shape of the switch cap 69 and linking lever 72, the main switch 6 remains in this on position. If the flow of current is not excessively large, the bimetal bar 78 also remains in the position shown, and the compression spring 75 maintains the existing position of the reset lever 74.

If the current flow becomes excessive, the input terminal 76 overheats, causing the bimetal bar 78 to curl as shown in FIG. 26D. The end of the bimetal bar 78 then pushes upward against a tab at the end of the reset lever 74, causing the reset lever to rotate in the direction of arrow F, compressing the compression spring 75, allowing the linking lever 72 to slip off the stopper 74a, thereby breaking the contact between the movable arm 77 and output terminal 79. When the reset lever 74 is in this position, the main switch 6 cannot be turned on, regardless of the position of the switch cap 69.

The pin 73b at the end of the detent bar 73 is now free to move downward in the drawing, allowing the torsion spring 71a to rotate the switch cap 69 in the direction of arrow B. Upon cooling, the bimetal bar 78 straightens, and the compression spring 75 rotates the reset lever 74 in the direction of arrow C, causing the main switch to return to the normal off state shown in FIG. 26A.

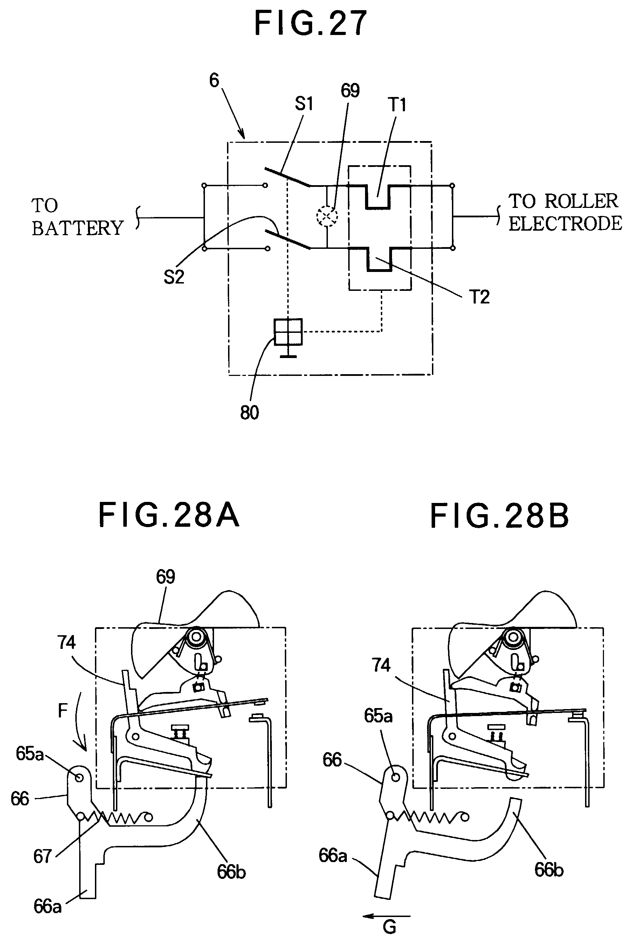

Although FIGs. 26A to 26D show only a single input terminal 76, movable arm 77, bimetal bar 78, and output terminal 79, the main switch 6 actually has two of each, as indicated schematically in FIG. 24. Referring to FIG. 27, the main switch 6 operates as if it comprised a pair of switches S1 and S2, both operated by the same switch cap 69, and a pair of overcurrent detectors T1 and T2, each actuating a switch release mechanism 80. The circuit including switch S1 and overcurrent detector T1 comprises one input terminal 76, movable arm 77, bimetal bar 78, and output terminal 79. The circuit including switch S2 and overcurrent detector T2 comprises another input terminal 76, movable arm 77, bimetal bar 78, and output terminal 79. The switch cap 69, linking lever 72, detent bar 73, reset lever 74, and compression spring 75 are shared by both circuits, the reset lever 74 and compression spring 75 constituting the switch release mechanism 80.

The two switches S1 and S2 are coupled in parallel and have equal current capacity, so during normal operation, current flow is divided equally between the two switching circuits. Each of the overcurrent detectors T1 and T2, that is, each of the two bimetal bars 78, actuates the switch release mechanism 80, namely the reset lever 74, upon detecting a current flow exceeding half the total permissible current flow. If the current flow through either one of the two switches S1 and S2 exceeds this value, both switches S1 and S2 are automatically turned off by the switch release mechanism 80.

The box detection lever 66 shown in FIG. 25 operates on the mechanism shown in FIGs. 26A to 26D as follows.

Referring to FIG. 28A, if the tray 5 is open or the waste box 51 is not installed, the bias spring 67 pulls the sensing end 66a of the box detection lever 66 toward the right in the drawing, causing the box detection lever 66 to pivot around the pin that fits into the hole 65a in the switch bracket. The pushing end 66b of the box detection lever 66 then pushes against the reset lever 74, which rotates in the direction of arrow F, breaking the electrical circuit between the input and output terminals of the main switch 6, even if the switch cap 69 is in the on position.

When the tray 5 is closed and the waste box 51 is installed, the waste box pushes the sensing end 66a of the box detection lever 66 in the direction of arrow G in FIG. 28B, forcing the box detection lever to pivot in this direction around the pin in hole 65a. The pushing end 66b of the box detection lever 66 then moves away from the reset lever 74, allowing the main switch 6 to be turned on.

Referring again to FIG. 24, the circuits on the control board 14 include a motor control circuit 81, an air-pump driver circuit 82, a battery charging control circuit 83, and a voltage-checking circuit 84. The motor control circuit 81 drives the motor 15b for a period of eight seconds, beginning when the limit switch 37 indicates that the float 4 has been depressed. The motor control circuit 81 comprises a resistor-capacitor integrating circuit (not visible) that measures the length of the eight-second interval. The air-pump driver circuit 82 drives the air pump 13 at a constant speed while the air pump is switched on by the sub-switch 6b. The battery charging control circuit 83 charges the battery 10 by means of an adapter 550 that plugs into the adapter socket 14b and a commercial alternating-current (AC) outlet (not visible). The voltage-checking circuit 84 detects the status of the battery 10 and drives the LED lamps 14a to inform the user of the remaining charge.

Next, the overall operation of the needle disposal apparatus will be described.

When the user moves the main switch 6 to the on position, the sub-switch 6b also turns on, and the air-pump driver circuit 82 begins driving the air pump 13. Air is thereby pumped through the entrance chamber 11a, melting chamber 12a, and waste box 51, which are held tightly together as described above to form a closed air passage. In addition, the main switch 6 connects the first roller electrode 18 to the battery 10, thereby creating a potential difference between the first roller electrode 18 and second roller electrode 19.

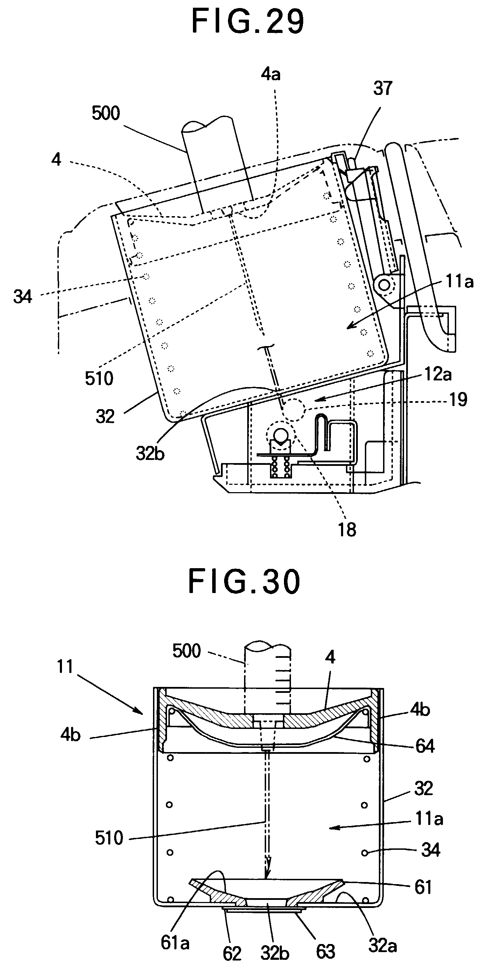

To dispose of a hypodermic needle 510, the user inserts the needle, attached to the hypodermic syringe 500, through the needle insertion hole 4a in the float 4, as shown in FIG. 29, and presses the syringe 500 and float 4 down against the forces exerted by compression of the compression spring 34 and compression of air in the entrance chamber 11a. The limit switch 37 detects the depression of the float 4, and notifies the motor control circuit 81, which begins driving the motor 15b that turns the first roller electrode 18 and second roller electrode 19.

As the float 4 is pressed down, the tip of the needle 510 moves through the hole 32b in the bottom of the float guide 32, passes into the melting chamber 12a, and reaches first the surface of the second roller electrode 19, then the surface of the first roller electrode 18. At this point, a short circuit is formed from the positive terminal of the battery 10 through the main switch 6, first roller electrode 18, needle 510, and second roller electrode 19 to the negative terminal of the battery 10. Flow of current through this short circuit heats and melts the tip of the needle 510. As the user continues to press down, more of the needle 510 is forced into the space between the first and second roller electrodes 18 and 19 and melted, until nearly all of the needle has been melted. The melted metal drops into the waste box 51.

As the needle 510 is being melted, fluids such as blood that remain in the needle are burnt or vaporized, creating odors that are carried by the continuing flow of air through the melting chamber 12a and waste box 51 to the odor filter 52. The odor filter 52 absorbs the odors, so that the air vented from the exhaust chamber 55b of the air pump 13 is substantially odor-free.

After pressing the syringe 500 and float 4 down as far as possible, the user withdraws the syringe, to which only the blunted and sterilized stump of the needle is now attached. The syringe and needle stump can be handled or disposed of without risk.

After the syringe 500 has been removed, the air pump 13 continues to pump air through the entrance chamber 11a, melting chamber 12a, and waste box 51, thereby removing any lingering odors from these locations. Due to the vigorous pumped air flow, and the airtight fit between the guide assembly 11, roller electrode assembly 12, and waste box 51, substantially all odors are removed by the odor filter 52 before they have a chance to seep into the space inside the top cover 2, middle cover 3, and base frame 9, or escape through the needle insertion hole 4a into the surrounding environment. The airflow is maintained until the user moves the main switch 6 to the off position.

The invented needle disposal apparatus can accordingly be used with substantially complete freedom from unpleasant odors, a significant advantage for medical personnel and patients alike.

A further advantage is that a person who opens the tray 5 to remove the waste box 51 can do so without being greeted by unpleasant odors issuing from the interior of the needle disposal apparatus, or the interior of the waste box, and the contents of the waste box can be disposed of without creating odor problems.

Another advantage is that the box detection lever 66 disables the main switch 6 when the waste box 51 is not installed or the tray 5 is not properly closed. The needle disposal apparatus accordingly never operates in a configuration that would permit odors to escape.

A related advantage is that the box detection lever 66 operates mechanically, without requiring additional electrical contacts inside the main switch 6, which would increase the cost of the main switch and lead to electrical loss (line drop).

The provision of two switching circuits S1 and S2 also reduces the cost of the main switch 6 by reducing the amount of current that each circuit has to switch, thus reducing the necessary size of the input terminals 76, connecting terminals 77, bimetal bars 78, and output terminals 79, while still providing sufficient current capacity to melt even large hypodermic needles.

The provision of two overcurrent detectors T1 and T2, that is, of two bimetal bars 78, enhances the reliability and safety of the main switch 6, because either one of the two overcurrent detectors T1 and T2 can shut the current off completely if the current flow becomes dangerously high.

Next, a second embodiment will be described. The second embodiment differs from the first in regard to the structure of the guide assembly.

Referring to FIG. 30, the guide assembly 11 of the second embodiment has a funnel 61 resting on the closed end 32a of the float guide 32, centered above the hole 32b in the closed end. An insulating sheet 62 is attached to the underside of the float guide 32 around the hole 32b. A shutter 63 is attached to the insulating sheet 62. A spring cap 64 is attached to the top of the compression spring 34, below the float 4.

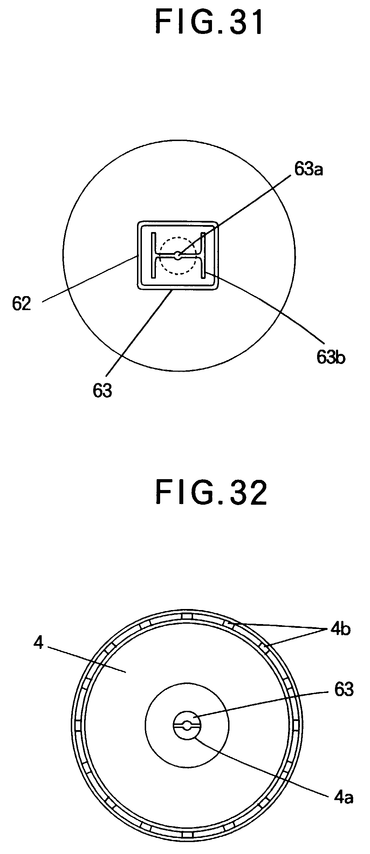

Referring to FIG. 31, the shutter 63 has a small central hole 63a disposed between two flaps defined by an H-shaped slot 63b. The insulating sheet 62 has a larger central hole, indicated by the dotted line. The shutter 63 is made of a flexible, heat-resistant material such as stainless spring steel, which is not harmed by flying drops of molten metal produced during the melting of a needle. The shutter 63 prevents such drops of molten metal from entering the entrance chamber 11a.

Referring again to FIG. 30, the funnel 61 has an inner surface 61a that is wide enough to catch drops of fluid that might fall from the tip of the needle 510 when the needle is first inserted into the guide assembly 11. Any such drops of fluid are channeled by the funnel 61 into the melting chamber 12a below, and eventually fall into the waste box 51.

The compression spring 34 and spring cap 64 both comprise electrically conducting metal materials, the compression spring 34 being made of, for example, stainless spring steel. The spring cap 64 is electrically coupled to the compression spring 34, which is electrically coupled to the float guide 32, which is electrically coupled to the supporting bracket 33, main bracket 17, middle cover 3, and base frame 9. The spring cap 64 has a central hole (not visible) for passage of the needle 510.

A plurality of air channels 4b are provided in the outer surface of the float 4, permitting air to move into and out of the entrance chamber 11a even when a hypodermic syringe 500 is inserted in the needle insertion hole 4a of the float. These air channels 4b are best seen in the top view in FIG. 32.

Other components of the second embodiment are the same as in the first embodiment. The floor and walls of the waste box 51 are made of an electrically conducting material. The tray 5 is also made of electrically conductive materials, so that the waste box 51 is electrically coupled to the base frame 9 and main bracket 17.

Next, the operation of the second embodiment will be described.

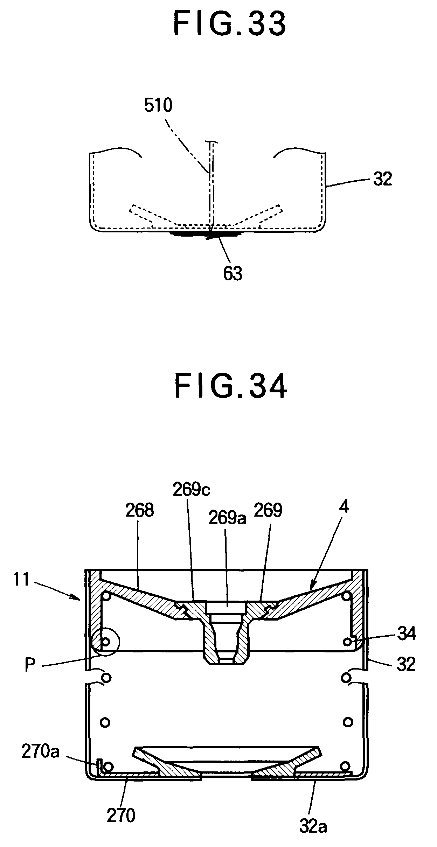

When a hypodermic syringe 500 is inserted in the second embodiment and pushed downward, the downward motion is resisted by the compression spring 34, but is not greatly resisted by the compression of air in the entrance chamber 11a, because the air flows outward through the air channels 4b. When the tip of the needle 510 reaches the shutter 63, the shutter is pushed open slightly, as indicated in FIG. 33. The flexible flaps of the shutter 63 guide the needle tip toward the central hole 63a, so that the needle 510 passes through this hole 63a; thus the shutter 63 needs to open only slightly. The needle tip enters the melting chamber 12a and is melted as in the first embodiment.

During the melting process, the needle 510 is enclosed on all sides by electrically conducting materials, namely the spring cap 64, the float guide 32, the electrode bracket 21 and back plate 24 of the roller electrode assembly 12, and the waste box 51, which act as a shield that absorbs electromagnetic noise. In particular, the spring cap 64 prevents the escape of electromagnetic interference through the float 4. The insulating sheet 62 prevents high voltages from being conducted to the float guide 32, and also prevents electromagnetic noise from being conducted to the float guide. The middle cover 3 and base frame 9 provide further shielding against electromagnetic noise.

When the user withdraws the syringe 500 at the end of the melting process, as the float 4 rises, air enters the entrance chamber 11a through the air channels 4b. The shutter 63 closes, except for the central hole 63a and H-shaped slot 63b, so little or no air flows from the melting chamber 12a through the shutter 63 into the entrance chamber 11a. The air pump 13 maintains a flow of air in the opposite direction, from the entrance chamber 11a into the melting chamber 12a. The float 4 is thus permitted to rise quickly without pumping odors from the melting chamber 12a into the entrance chamber 11a.

One advantage of the second embodiment is that the interior of the guide assembly 11 is kept clean and odor-free by the funnel 61 and shutter 63.

Another advantage is that, as odors are confined to the melting chamber 12a and waste box 51 by the shutter 63, the odor filter 52 is able to operate even more efficiently than in the first embodiment.

Still another advantage is that, because air can escape from the entrance chamber 11a through the air channels 4b as the float 4 is pressed downward, compressed air is not forced into the melting chamber 12a and waste box 51, and any odors present in these locations are not pumped out into the surrounding environment.

Yet another advantage is that the air channels 4b allow the float 4 to move upward and downward more quickly and easily than in the first embodiment, making the needle disposal apparatus more convenient to use.

Next, a third embodiment will be described. The third embodiment differs from the preceding embodiments mainly in regard to the structure of the float.

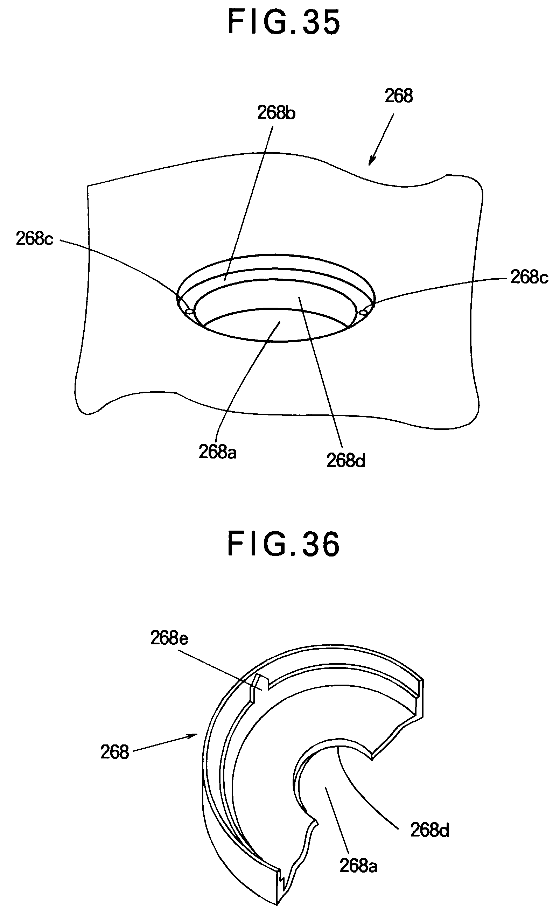

Referring to FIG. 34, the float 4 in the third embodiment comprises a slider 268 and a replaceable syringe guide cap 269. Referring to FIG. 35, the slider 268 has a central hole 268a surrounded by a shallow step 268b with two hemispherical indentations 268c. These indentations 268c are disposed on an imaginary circle concentric with the central hole 268a, on opposite sides of the central hole. The inside surface 268d of the central hole 268a is tapped with screw threads (not visible). Referring to FIG. 36, at least one triangular-tipped projection 268e is provided on the underside of the slider 268. The projection 268e is disposed near the outside perimeter of the slider 268, at the location indicated by the circle P in FIG. 34. All such projections 268e, if there are more than one, are disposed on another imaginary circle concentric with the central hole 268a.

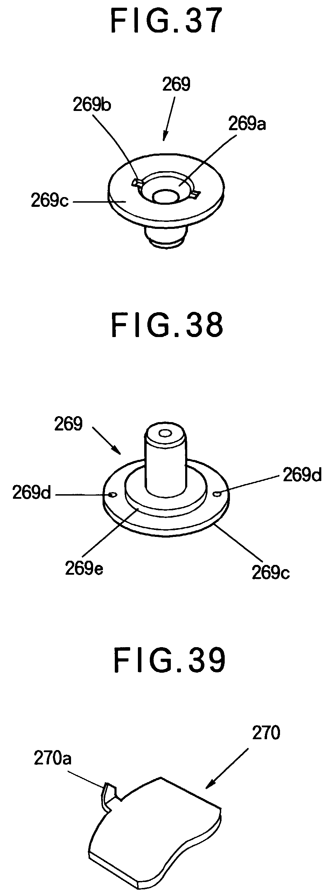

The syringe guide cap 269 has a central hole 269a surrounded by a flange 269c. Referring to FIG. 37, the flange 269c has a pair of notches 269b on opposite sides of the central hole 269a. Referring to FIG. 38, the underside of the flange 269c has a pair of hemispherical projections 269d that fit into the hemispherical indentations 268c of the slider 268, and a surface 269e with threads (not visible) that engage the threads on the inside surface 268d of the slider.

Referring again to FIG. 34, a stopper plate 270 is attached to the closed end 32a of the float guide 32. The stopper plate 270 has a funnel shape to catch falling drops of fluid as in the second embodiment. Referring to FIG. 39, the stopper plate 270 has at least one triangular-tipped projection 270a, which is aligned with the concentric circle on which the triangular-tipped projection 268e of the slider 268 is disposed.

The shape of the central hole 269a of the syringe guide cap 269 is designed to guide and hold the hypodermic syringe and needle when the needle is inserted in the needle disposal apparatus. The needle disposal apparatus is preferably provided with a plurality of syringe guide caps 269, designed to fit different types of syringes and needles. To replace one syringe guide cap 269 with another, the user employs a coin or other tool inserted in the notches 269b to unscrew the old syringe guide cap from the slider 268. Before unscrewing, the user pushes the float 4 down to the bottom of the guide assembly 11. When the float 4 is pushed down, the triangular-tipped projection 268e on the slider 268 may strike the triangular-tipped projection 270a of the stopper plate 270, but the two triangular tips will slide past each other with a slight rotation of the slider.

When the float 4 has been pushed all the way down, the user begins turning the syringe guide cap 269. At first, the slider 268 may also turn, but this turning of the slider brings the triangular-tipped projection 268e of the slider into contact with the triangular-tipped projection 270a of the stopper plate 270, at which point the slider stops turning and the syringe guide cap 269 begins to unscrew from the slider. A certain force must be applied to lift the hemispherical projections 269d out of the hemispherical indentations 268c, but thereafter, the syringe guide cap 269 unscrews easily.

The new syringe guide cap 269 is installed in the same way, by placing the syringe guide cap in the central hole 268a of the slider 268, pushing the float 4 all the way down, then turning the syringe guide cap with a coin or other tool. When the new syringe guide cap 269 is completely screwed into place, the hemispherical projections 269d protrude into the hemispherical indentations 268c, preventing unintended loosening of the syringe guide cap during use.

After the appropriate syringe guide cap 269 is installed, when a hypodermic syringe and needle are inserted into the syringe guide cap, the needle is guided straight toward the entrance to the melting chamber 12a, and the needle accurately follows the path marked X in FIG. 7, ensuring that the needle tip correctly enters the space between the roller electrodes 18 and 19. When the float 4 is pushed down by the syringe, the triangular-tipped projections 268e and 270a may collide, but they will slide past each other with a slight rotation of the float 4.

Aside from the structure of the float 4 and the stopper plate 270, the third embodiment is similar to the first embodiment and operates in the same way.

The third embodiment is easy to use, because the syringe guide cap 269 guides the needle in the proper direction.

Since the syringe guide cap 269 is replaceable, the third embodiment can be used with a wide variety of hypodermic syringes and needles, including both large and small needles.

In the embodiments described above, the tray 5 was withdrawn to permit loading of the waste box 51 into the waste-box holder 44, but the structure of the tray can be varied to permit loading when the orientation of the tray or waste-box holder is changed.

The air pump 13 can be driven at a variable speed: for example, at one speed during the melting of a needle, and another speed before and after the melting operation.

The main switch 6 is not limited to two parallel switching circuits as shown in FIGs. 24 and 28, but can comprise n parallel switching circuits, where n is an integer greater than two. Each switching circuit carries 1/n of the total current, and each switching circuit has an overcurrent detector that detects current flow exceeding 1/n of the maximum permissible current.

In the second embodiment, the spring cap 64 can be omitted if the float 4 is made of an electrically conducting material.

The compression spring 34 in the second embodiment can be made of phosphor bronze for improved electrical conductivity.

The air channels in the second embodiment may be disposed in the body of the float 4 instead of being disposed around the perimeter of the float. Alternatively, air channels can be provided near the bottom of the float guide 32, instead of in the float.

Those skilled in the art will recognize that further variations are possible within the scope claimed below.

## Claims

1. A needle disposal apparatus, comprising:
an electrode assembly (12) having a first enclosing portion (21, 24) forming a melting chamber (12a), and electrodes (18, 19), disposed in said melting chamber, for melting a hypodermic needle (510) by passage of electrical current through said hypodermic needle;
a guide assembly (11) disposed in contact with said electrode assembly (12), having a second enclosing portion (32) forming an entrance chamber (11a), and means (4) for guiding said hypodermic needle (510) through said entrance chamber into said melting chamber (12a), said second enclosing portion making tight contact with said first enclosing portion (21, 24), so that said entrance chamber and said melting chamber form an airtight passage;
a waste box (51) having a top opening (51a'') and a vent opening (52c), said waste box being disposed below said electrode assembly (12) so that the melted hypodermic needle (510) drops through said top opening into said waste box;
a tray (5) holding said waste box (51) in tight contact with said electrode assembly (12), so that said waste box and said melting chamber (12a) form another airtight passage;
an odor filter (52) for absorbing odors created when said hypodermic needle (510) is melted; and
an air pump (13) coupled to said odor filter (52) and the vent opening (52c) of said waste box (51), drawing air through said entrance chamber (11a), said melting chamber (12a), said waste box, and said odor filter, thereby removing said odors from said melting chamber and said waste box.

2. The needle disposal apparatus of claim 1, wherein said odor filter (52) is disposed in said waste box (51), facing said vent opening (52c).

3. The needle disposal apparatus of claim 2, wherein said waste box (51) has a partition (51g) dividing said waste box into a waste chamber (51e) and a filter chamber (51f), the melted hypodermic needle (510) falling into said waste chamber, said odor filter (52) being disposed in said filter chamber, said partition being separated from said odor filter by a certain space, said partition having an upper opening (51h) for flow of air from said waste chamber into said filter chamber.

4. The needle disposal apparatus of claim 1, wherein said tray (5) comprises a first mechanism (40) for moving said tray to a first position, in which said waste box (51) is held in tight contact with said electrode assembly (12), and a second position, in which said waste box is removable from said tray.

5. The needle disposal apparatus of claim 4, wherein said tray (5) further comprises a second mechanism automatically raising said waste box (51) when said tray is moved from said second position to said first position, and automatically lowering said waste box when said tray is moved from said first position to said second position.

6. The needle disposal apparatus of claim 5, wherein said second mechanism comprises:
a waste-box holder (44) holding said waste box (51);
a spring (50) urging said waste-box holder (44) upward;
a collar (47) attached to said waste-box holder (44); and
a sliding arm (45) having a slot (45f) with a sloping edge (45j) engaging said collar (47), forcing said collar downward when said sliding arm is moved in a certain direction.

7. The needle disposal apparatus of claim 6, further comprising a locking arm (48) attached to said waste-box holder (44), locking said tray (5) in said first position when said waste-box holder moves upward, and unlocking said tray when said waste-box holder moves downward.

8. The needle disposal apparatus of claim 1, further comprising an air-pump driver circuit (82) activating said air pump (13) before, while, and after said hypodermic needle (510) is melted by said electrode assembly (12).

9. The needle disposal apparatus of claim 1, wherein said electrodes (18, 19) have a roller shape, further comprising a motor (15b) rotating said electrodes while said hypodermic needle (510) is being melted.

10. The needle disposal apparatus of claim 1, wherein said means (4) for guiding comprises a float forming a movable end of said entrance chamber (11a), said float having a hole (4a) for insertion of said hypodermic needle (510), at least one of said float and said second enclosing portion (32) having air channels (4b) permitting air to move in and out of said entrance chamber as said float is moved.

11. The needle disposal apparatus of claim 1, further comprising a shutter (63) disposed between said entrance chamber (11a) and said melting chamber (12a), said shutter opening when said hypodermic needle (510) presses against said shutter.

12. A needle disposal apparatus, comprising:
an electrode assembly (12) having a melting chamber (12a) with electrodes (18, 19) for melting a hypodermic needle (510) by passage of electrical current through said hypodermic needle;
a guide assembly (11) disposed in contact with said electrode assembly (12), having an entrance chamber (11a) and means (4) for guiding said hypodermic needle (510) through said entrance chamber into said melting chamber (12a); and
a plurality of covers (32, 64) surrounding said entrance chamber (11a), said covers being formed from electrically conducting materials and being mutually interconnected, thus acting as shields for electromagnetic noise generated when said hypodermic needle (510) is melted.

13. A needle disposal apparatus, comprising:
an electrode assembly (12) having a first enclosing portion (21, 24) forming a melting chamber (12a), and electrodes (18, 19), disposed in said melting chamber, for melting a hypodermic needle (510) by passage of electrical current through said hypodermic needle;
a guide assembly (11) disposed in contact with said electrode assembly (12), having a second enclosing portion (32) forming an entrance chamber (11a) communicating with said melting chamber (12a), and means (4, 34, 64) for guiding said hypodermic needle (510) through said entrance chamber into said melting chamber; and
a waste box (51) disposed below said electrode assembly (12), having an open top (51a'') opening into said melting chamber (12a), and closed sides and bottom, for receiving the melted hypodermic needle (510);
wherein said first enclosing portion (21, 24), said second enclosing portion (32), said waste box (51), and at least part of said means (4, 34, 64) for guiding are formed from electrically conducting materials that act as shields for electromagnetic noise generated when said hypodermic needle (510) is melted.

14. A needle disposal apparatus, comprising:
an electrode assembly (12) with electrodes (18, 19) for melting a hypodermic needle (510) by passage of electrical current through said hypodermic needle;
a power supply (10) coupled to said electrode assembly (12), supplying said electrical current to said electrodes (18, 19);
a switch (6) coupled between said electrode assembly (12) and said power supply (10), switching said electrical current on and off;
a waste box (51) disposed below said electrode assembly (12), for receiving the melted hypodermic needle (510);
a tray (5) holding said waste box (51) below said electrode assembly (12), said waste box being removable from said tray; and
a means for detecting whether said waste box (51) is held below said electrode assembly (12) by said tray (5), and resetting said switch (6), thereby preventing said electrical current from being supplied to said electrodes (18, 19), when said waste box (51) is not held below said electrode assembly (12) by said tray.

15. The needle disposal apparatus of claim 14, wherein:
said switch (6) has a first spring (75) and a reset lever (74), said reset lever being movable to a first position permitting said switch to be turned on and a second position preventing said switch from being turned on, said first spring urging said reset lever toward said first position; and
said means for detecting comprises a second spring (67) and a box detection lever (66), said box detection lever being movable to a third position contacting said waste box (51) when said waste box is held below said electrode assembly (12) by said tray (5), and a fourth position forcing said reset lever (74) to said second position, said second spring urging said box detection lever toward said fourth position.

16. A needle disposal apparatus, comprising:
an electrode assembly (12) with electrodes (18, 19) for melting a hypodermic needle (510) by passage of electrical current through said hypodermic needle;
a power supply (10) coupled to said electrode assembly (12), supplying said electrical current to said electrodes (18, 19); and
a plurality of switches (S1, S2) coupled in parallel between said electrode assembly (12) and said power supply (10), switching said electrical current on and off.

17. The needle disposal apparatus of claim 16, further comprising a plurality of overcurrent detectors (T1, T2) coupled to respective switches (S1, S2), each one of said overcurrent detectors switching all of said switches off if current exceeding a predetermined limit value is detected.

18. The needle disposal apparatus of claim 17, wherein said switches (S1, S2) have equal current capacities, and said predetermined limit value is 1/n of a total permissible current value, where n is an integer said switches are n in number.

19. A needle disposal apparatus, comprising:
an electrode assembly (12) having a melting chamber (12a) with electrodes (18, 19) for melting a hypodermic needle (510) by passage of electrical current through said hypodermic needle; and
a guide assembly (11) disposed in contact with said electrode assembly (12), having a syringe guide cap (269) for holding said hypodermic needle (510), and a slider (268) in which said syringe guide cap is removably mounted, said slider moving to guide said hypodermic needle (510) into said melting chamber (12a).

20. The needle disposal apparatus of claim 19, wherein said syringe guide cap (269) screws into said slider (268).

21. The needle disposal apparatus of claim 20, wherein said slider (268) has a projection (268e) for stopping rotation of said slider when said syringe guide cap (269) is being screwed into said slider.
